# FASCICULE DE BREVET EUROPEEN

(11) **EP 0 739 625 B1**
(45) Date de publication et mention de la délivrance du brevet: **20.06.2001**
(21) Numéro de dépôt: 95400922.1
(22) Date de dépôt: 25.04.1995
(51) Int. Cl.: A61K 7/50, A61K 7/48, A61K 7/06

(54) **Compositions pour le lavage et le traitement des cheveux et de la peau à base de céramide et de polymères à groupement cationiques**
Haut- und Haarwaschpflegemittel auf Basis von Ceramiden und kationischen Polymeren
Washing and treating composition for skin and hair based on ceramides and cationic polymers

(43) Date de publication de la demande: 30.10.1996
(73) Titulaire: L'OREAL, 75008 Paris (FR)
(72) Inventeur: Cauwet, Danièle, F-75011 Paris (FR); Dubief, Claude, F-78150 Le Chesnay (FR); Beauquey, Bernard, F-92111 Clichy (FR)
(74) Mandataire: Casalonga, Axel

(56) Documents cités:
- EP-A- 0 278 505
- WO-A-93/02656
- WO-A-94/03151

## Description

La présente invention est relative à de nouvelles compositions de lavage et de traitement des cheveux et/ou de la peau, contenant au moins un agent tensio-actif anionique, un agent tensio-actif amphotère, un céramide et/ou glycocéramide et un polymère à groupements cationiques, aux procédés de traitement cosmétiques mettant en oeuvre de telles compositions.

Les shampooings ayant un effet conditionneur des cheveux sont bien connus dans l'état de la technique et nombre d'entre eux reposent sur l'utilisation de polymères cationiques.

Ces shampooings, bien qu'ils présentent de façon générale de bonnes performances, sont cependant encore considérés par l'homme de l'art comme insatisfaisants, notamment au niveau du démêlage des cheveux humides, du toucher et de l'aspect esthétique des cheveux séchés, en particulier lors de superpositions de différents traitements.

Les inventeurs ont découvert qu'en mettant en oeuvre un shampooing contenant des agents tensio-actifs anioniques, des agents tensio-actifs amphotères, des polymères à groupements cationiques, un céramide et/ou un glycocéramide, on constatait de façon surprenante une amélioration importante des performances au niveau cosmétique.

Les céramides ou glycocéramides sont connus en eux-mêmes et ont déjà été préconisés dans le brevet FR-A-2 673 179, notamment dans des shampooings. Les shampooings diffèrent cependant des shampooings conformes à l'invention par le fait qu'ils ne contiennent ni agents tensio-actifs amphotères, ni polymères cationiques, et ne conduisent pas aux propriétés constatées avec les shampooings conformes à l'invention.

La demande WO 93/02656 a pour objet des dispersions cationiques de céramide et/ou de glycocéramide. Ce document ne met cependant pas en oeuvre des polymères cationiques.

On connaît également par le brevet EP-A-278 505 des compositions pouvant être des shampooings, contenant un céramide ou glycocéramide et au moins un ester de cholestérol dans un véhicule cosmétique approprié. Il n'est jamais envisagé dans ce document l'utilisation d'agents tensio-actifs amphotères.

La demanderesse a plus particulièrement constaté une synergie du démêlage obtenue grâce à l'association d'un polymère à groupements cationiques, d'un céramide et/ou glycocéramide, dans un shampooing contenant au moins un tensio-actif anionique et au moins un tensio-actif amphotère ou zwitterionique. Il a également été constaté un dépôt amélioré du céramide ou du glycocéramide sur les cheveux.

On appelle "effet conditionneur" ou "conditionnement", selon l'invention, un effet conférant aux cheveux de bonnes propriétés de démêlage, tant à l'état humide qu'à l'état sec, de bonnes propriétés au toucher des cheveux séchés et un aspect esthétique, signe de vitalité.

Un objet de l'invention est donc constitué par une composition de lavage et de traitement des cheveux et/ou de la peau, à base d'agents tensio-actifs anioniques, amphotères ou zwitterioniques, d'un polymère à groupements cationiques et d'un céramide et/ou d'un glycocéramide.

Un autre objet de l'invention est constitué par un procédé de traitement cosmétique des cheveux et/ou de la peau, mettant en oeuvre une telle composition.

D'autres objets de l'invention apparaîtront à la lecture de la description et des exemples qui suivent.

La composition de lavage et de traitement cosmétique de la peau et/ou des cheveux, conforme à l'invention, est essentiellement caractérisée par le fait qu'elle contient dans un milieu cosmétiquement acceptable :
- au moins un agent tensio-actif anionique,
- au moins un agent tensio-actif amphotère ou zwitterionique, les agents tensio-actifs étant présents dans une proportion au moins égale à 4% en poids par rapport au poids de la composition,
- au moins un polymère à groupements cationiques,
   et
- au moins un céramide et/ou glycocéramide.

Les agents tensio-actifs anioniques et amphotères ou zwitterioniques sont choisis parmi les agents tensio-actifs présentant des propriétés détergentes. Ils sont utilisés dans des proportions suffisantes pour conférer à la composition des propriétés détergentes.

Parmi les agents tensio-actifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkylétherphosphates; les acylsarcosinates, les acyliséthionates, les N-acyltaurates.

Le radical alkyle ou acyle de ces différents composés est généralement constitué par une chaîne carbonée comportant de 12 à 20 atomes de carbone.

Le radical aryle est généralement constitué par un groupement phényle ou benzyle.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 20 atomes de carbone.

On peut également utiliser des agents tensio-actifs classés généralement dans la famille des agents faiblement anioniques tels que les acides alkyl D-galactoside uroniques et leurs sels et les acides d'éthers carboxyliques polyoxyalkylénés et leurs sels, et en particulier ceux comportant 2 à 50 groupements oxyde d'éthylène.

On peut plus particulièrement citer les acides et sels d'acides éthers carboxyliques polyoxyéthylénés de formule :

R₁(OCH₂CH₂)ₙOCH₂COOA

dans laquelle R₁ est un radical alkyl ou alkaryl et n a une valeur moyenne comprise entre 2 et 20, le radical alkyl ayant entre 6 et 20 atomes de carbone, aryl désignant de préférence phényle.

A désigne hydrogène, un métal alcalin ou alcalino-terreux, une amine ou un ammonium.

On peut citer plus particulièrement les produits vendus sous la dénomination AKYPO par la Société CHEM'Y.

Les agents tensio-actifs amphotères ou zwitterioniques sont notamment choisis parmi les dérivés d'amines secondaires ou tertiaires aliphatiques dans lesquels le radical aliphatique est une chaîne linéaire ou ramifiée comportant 8 à 18 atomes de carbone et contenant au moins un groupement anionique hydrosolubilisant tel que carboxylate, sulfonate, sulfate, phosphate ou phosphonate.

On peut également citer les alkyl(C₈-C₂₀)bétaïnes, les sulfobétaïnes, les alkyl(C₈-C₂₀)amidoalkyl(C₁-C₆)bétaïnes ou les alkyl (C₈-C₂₀)amidoalkyl(C₁-C₆)sulfobétaïnes.

Parmi les dérivés d'amines, on peut citer les produits vendus sous la dénomination MIRANOL tels que décrits dans les brevets US-A-2 528 378 et US-A-2 781 354 et classés dans le Dictionnaire CTFA, Sème édition, 1993, sous les dénominations "Disodium coco-amphodiacétate" et "Disodium amphocarboxypropionate".

Les polymères à groupements cationiques sont connus en eux-mêmes et sont plus particulièrement choisis parmi les polymères cationiques, c'est-à-dire contenant uniquement des groupements cationiques, les polymères des protéines quaternisées et les polymères amphotères. Ces polymères sont des polymères "substantifs" qui peuvent être révélés à l'aide du colorant acide Red 80 selon RICHARD J. CRAWFORD, Journal of the Society of Cosmetic Chemists, 1980, 31 (5), pages 273 à 278.

Ces polymères sont choisis plus particulièrement parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1.000 et 3.000.000.

Parmi ces polymères, on peut citer plus particulièrement les protéines quaternisées, les polymères de la famille des polyamines, des polyaminoamides, des polyammonium quaternaires, les polysiloxanes cationiques.
**A.** Les protéines quaternisées sont en particulier des polypeptides modifiés chimiquement et portant en bout de chaîne ou greffés sur celle-ci, des groupements ammonium quaternaires. Parmi ces protéines, on peut citer notamment :
   - les hydrolysats de collagène portant des groupements triéthylammonium tels que les produits vendus sous la dénomination "QUATPRO E" par la Société MAYBROOK et dénommés dans le dictionnaire CTFA "Triéthonium Hydrolyzed Collagen Ethosulfate".
**B.** Les polymères de la famille des polyamines, polyaminoamides ou polyammonium quaternaires, utilisables conformément à la présente invention, sont décrits en particulier dans les brevets français de la demanderesse n° 2.505.348 ou 2.542.997.

Parmi ces polymères, on peut citer :
(1) Les copolymères vinylpyrrolidone/acrylate ou méthacrylate de dialkylaminoalkyle quaternisés ou non, tels que les produits vendus sous la dénomination "GAFQUAT" par la Société GAF CORPORATION comme par exemple "GAFQUAT 734 ou 755", ou bien les produits dénommés "COPOLYMER 845, 958 et 937". Ces polymères sont décrits en détail dans les brevets français 2.077.143 et 2.393.573.
(2) Les dérivés d'éthers de cellulose comportant des groupements ammonium quaternaires décrits dans le brevet français 1.492.597 et en particulier les polymères commercialisés sous les dénominations "JR" (JR 400, JR 125, JR 30M) ou "LR" (LR 400, LR 30M) par la Société UNION CARBIDE CORPORATION. Les polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés par un monomère hydrosoluble d'ammonium quaternaire et décrits plus en détail dans le brevet américain 4.131.576 tels que les hydroxyalkylcelluloses comme les hydroxyméthyl-, hydroxyéthyl- ou hydroxypropylcellulose greffées par un sel de méthacryloyléthyltriméthylammonium, méthacrylamidopropyltriméthylammonium, diméthyldiallylammonium.
   Les produits commercialisés répondant à cette définition sont plus particulièrement les produits vendus sous les dénominations "CELQUAT L 200" et "CELQUAT H 100" par la Société NATIONAL STARCH.
(4) Les polysaccharides quaternisés décrits plus particulièrement dans les brevets américains 3.589.578 et 4.031.307 et plus particulièrement le produit commercialisé sous la dénomination "JAGUAR C 13 S" vendu par la Société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont décrits dans les brevets français 2.162.025 et 2.280.361.
(6) Les polyaminoamides solubles dans l'eau préparés en particulier par polycondensation d'un composé acide avec une polyamine. Ces polyaminoamides peuvent être réticulés par une épihalohydrine, un diépoxyde, un dianhydride, un anhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé; l'agent réticulant étant utilisé dans des proportions allant de 0,025 à 0,35 mole par groupement amine du polyaminoamide.
   Ces polyaminopolyamides peuvent être alcoylés ou s'ils comportent une ou plusieurs fonctions amines tertiaires, quaternisés. De tels polymères sont décrits en particulier dans les brevets français 2.252.840 et 2.368.508.
(7) Les dérivés de polyaminoamides résultant de la condensation de polyalcoylène polyamines avec des acides polycarboxyliques suivie d'une alcoylation par des agents bifonctionnels. On peut citer par exemple les polymères acide adipique-dialcoylaminohydroxyalcoyldialcoylène triamine dans lesquels le radical alcoyle comporte de 1 à 4 atomes de carbone et désigne de préférence méthyle, éthyle, propyle. De tels polymères sont décrits dans le brevet français 1.583.363.
   Parmi ces dérivés, on peut citer plus particulièrement les polymères acide adipique/diméthylaminohydroxypropyl/diéthylène triamine vendus sous la dénomination "CARTARETINE F, F₄ ou F₈" par la Société SANDOZ.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre la polyalkylène polyamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont décrits en particulier dans les brevets américains 3.227.615 et 2.961.347.
   Des polymères de ce type sont en particulier commercialisés sous la dénomination "HERCOSETT 57" par la Société HERCULES INCORPORATED ou bien sous la dénomination de "PD 170" ou "DELSETTE 101" par la Société HERCULES dans le cas du copolymère d'acide adipique/époxypropyl/diéthylène-triamine.
(9) Les cyclopolymères de méthyl diallyl amine ou de diméthyl diallyl ammonium ayant un poids moléculaire de 20 000 à 3 000 000 tels que les homopolymères comportant comme constituant principal de la chaîne des motifs répondant aux formules (II) ou (II') : 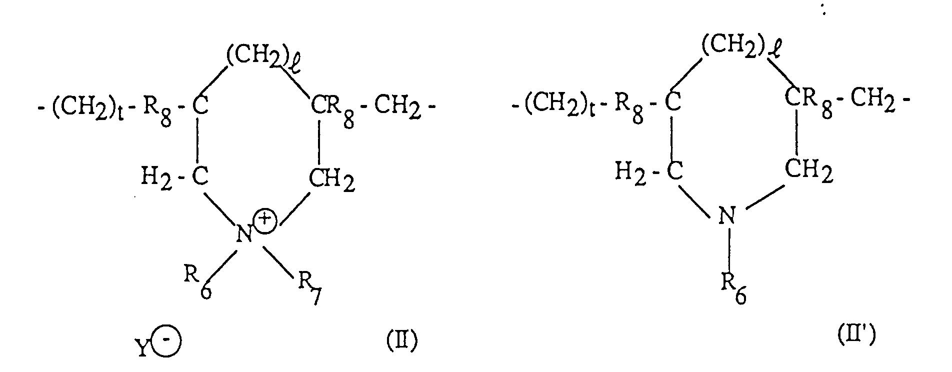
   ℓ et t sont égaux à 0 ou 1, et la somme ℓ+ t = 1;
   R₈ désigne hydrogène ou méthyle;
   R₆ et R₇ désignent, indépendamment l'un de l'autre, un groupement alkyle ayant de 1 à 22 atomes de carbone, un groupement hydroxyalkyle dans lequel le groupement alkyle a de préférence 1 à 5 atomes de carbone, un groupement amidoalkyle inférieur et où R₆ et R₇ peuvent désigner conjointement avec l'atome d'azote auquel ils sont rattachés, des groupements hétérocycliques tels que pipéridinyle ou morpholinyle, ainsi que les copolymères comportant des unités de formules (II) ou (II') et des unités dérivés d'acrylamide ou de diacétone acrylamide;
   Y^{⊖} est un anion tel que bromure, chlorure, acétate, borate, citrate, tartrate, bisulfate, bisulfite, sulfate, phosphate.

   Parmi les polymères définis ci-dessus, on peut citer plus particulièrement l'homopolymère de chlorure de diméthyldiallylammonium vendu sous la dénomination "MERQUAT 100", ayant un poids moléculaire inférieur à 100 000, le copolymère de chlorure de diméthyldiallylammonium et d'acrylamide ayant un poids moléculaire supérieur à 500 000 et vendu sous les dénominations "MERQUAT 550" et "S" par la Société MERCK.
   Ces polymères sont décrits plus particulièrement dans le brevet français 2.080.759, et son certificat d'addition n° 2.190.406.
(10) Le polymère de polyammonium quatenaire contenant des motifs récurrents répondant à la formule : 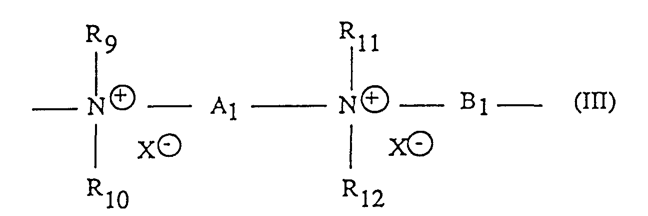 dans laquelle R₉ et R₁₀, R₁₁ et R₁₂ étant identiques ou différents, représentent des radicaux aliphatiques, alicycliques ou arylaliphatiques contenant de 1 à 20 atomes de carbone ou des radicaux hydroxy alkylaliphatiques inférieurs, ou bien R₉ et R₁₀ et R₁₁ et R₁₂ ensemble ou séparément, constituent avec les atomes d'azote auxquels ils sont rattachés, des hétérocycles contenant éventuellement un second hétéroatome autre que l'azote ou bien R₉, R₁₀, R₁₁ et R₁₂ représentent un radical alkyle en C₁-C₆ linéaire ou ramifié substitué par un groupement nitrile, ester, acyle, amide ou 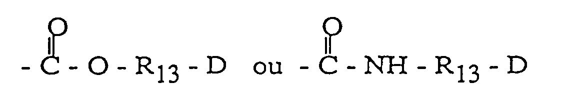 où R₁₃ est un alkylène et D un groupement ammonium quaternaire.
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X^{⊖} désigne un anion dérivé d'un acide minéral ou organique.
   A₁ et R₉ et R₁₁ peuvent former avec les deux atomes d'azote auxquels ils sont rattachés, un cycle pipérazinique; en outre, si A₁ désigne un radical alkylène ou hydroxyalkylène linéaire ou ramifié, saturé ou insaturé, B₁ peut également désigner un groupement

   (CH₂)ₙ - CO - D - OC - (CH₂)ₙ -

   dans lequel D désigne :
   a) un reste de glycol de formule : - O - Z - O -
      où Z désigne un radical hydrocarboné linéaire ou ramifié ou un groupement répondant aux formules : 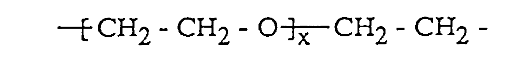 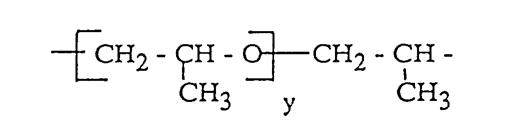 où x et y désignent un nombre entier de 1 à 4, représentant un degré de polymérisation défini et unique ou un nombre quelconque de 1 à 4 représentant un degré de polymérisation moyen;
   b) un reste de diamine bis-secondaire tel qu'un dérivé de pipérazine;
   c) un reste de diamine bis-primaire de formule :

      - NH - Y - NH -

      où Y désigne un radical hydrocarboné linéaire ou ramifié, ou bien le radical bivalent

      - CH₂ - CH₂ - S - S - CH₂ - CH₂ -
   d) un groupement uréylène de formule :

      - NH - CO - NH - ;

      X^{⊖} est un anion tel que chlorure ou bromure.

   Ces polymères ont une masse moléculaires généralement comprise entre 1000 et 100 000.
   Des polymères de ce type sont décrits en particulier dans les brevets français 2 320 330, 2 270 846, 2 316 271, 2 336 434, et 2 413 907 et les brevets US-A-2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.
(11) Les polymères de polyammonium quaternaires constitués de motifs de formule (IV) : 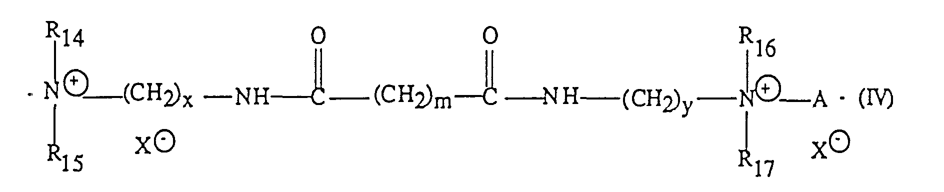 dans laquelle :
   R₁₄, R₁₅, R₁₆ et R₁₇, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à O ou un nombre entier compris entre 1 et 6, sous réserve que R₁₄, R₁₅, R₁₆ et R₁₇ ne représentent pas simultanément un atome d'hydrogène;
   x et y, identiques ou différents, sont des nombres entiers compris entre 1 et 6;
   m est égal à O ou à un nombre entier compris entre 1 et 34;
   x désigne un atome d'halogène;
   A désigne un radical d'un dihalogénure et représente de préférence

      - CH₂ - CH₂ - O - CH₂ - CH₂ -

   De tels composés sont décrits plus en détail dans la demande EP-A-122 324.
   On peut par exemple citer parmi ceux-ci, les produits "MIRAPOL A 15", "MIRAPOL AD1", "MIRAPOL AZ1", "MIRAPOL 175", vendus par la Société MIRANOL.
(12) Les homopolymères ou copolymères dérivés d'esters ou d'amides acrylique ou méthacrylique et comportant les motifs : 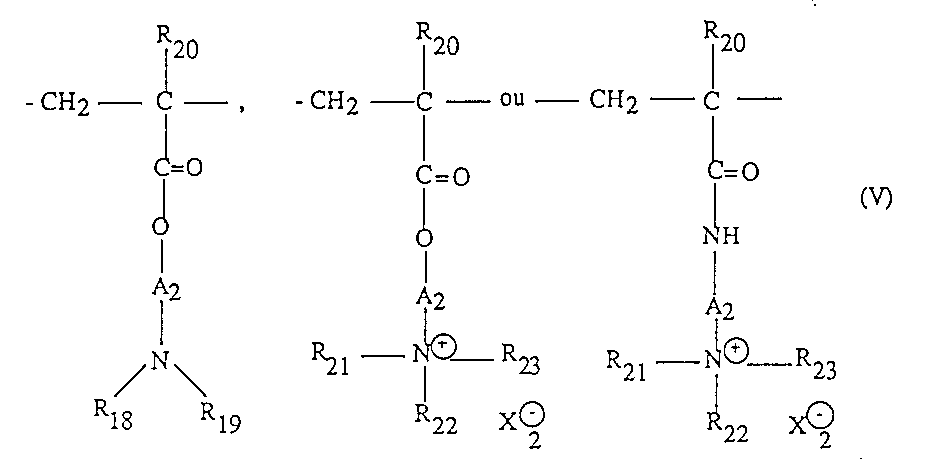 dans lesquels :
   R₂₀ désigne H ou CH₃;
   A₂ est un groupe alkyle linéaire ou ramifié de 1 à 6 atomes de carbone ou un groupe hydroxyalkyle de 1 à 4 atomes de carbone;
   R₂₁, R₂₂, R₂₃, identiques ou différents, représentent un groupe alkyle ayant de 1 à 18 atomes de carbone ou un radical benzyle;
   R₁₈ et R₁₉ représentent hydrogène ou un groupe alkyle ayant de 1 à 6 atomes de carbone;
   X^{⊖}₂ désigne un anion méthosulfate ou un halogénure tel que chlorure ou bromure.

   Le ou les comonomères utilisables appartiennent à la famille des acrylamide, méthacrylamide, diacétone acrylamide, acrylamide et méthacrylamide substitués à l'azote par des alkyles inférieurs, des acides acrylique ou méthacrylique ou leurs esters, la vinylpyrrolidone, des esters vinyliques.
   Parmi ces composés, on peut citer le copolymère d'acrylamide et de diméthylaminoéthyl méthacrylate quaternisé au sulfate de diméthyle et vendu sous la dénomination "HERCOFLOC" par la Société HERCULES, le copolymère d'acrylamide et de chlorure de méthacryloyloxyéthyltriméthylammonium - décrit dans la demande de brevet EP-A-80976 - et vendu sous la dénomination "BINA QAT P100" par la Société CIBA GEIGY, ou encore le poly(chlorure de méthacrylamidopropyltriméthylammonium) vendu sous la dénomination "POLYMAPTAC" par la Société TEXACO CHEMICALS, le méthacryloyloxyéthyltriméthylammonium méthosulfate et son copolymère avec l'acrylamide vendus sous la dénomination "RETEN" par la Société HERCULES.
(13) Les polymères quaternaires de vinylpyrrolidone et de vinylimidazole tel que par exemple les produits commercialisés sous les dénominations "LUVIQUAT FC 905, FC 550 et FC 370" par la Société BASF.
(14) Les polyamines comme le Polyquart H vendu par HENKEL, référencé sous le nom de "POLYETHYLENEGLYCOL (15) TALLOW POLYAMINE" dans le dictionnaire CTFA.
(15) Les polymères réticulés de chlorure de méthacryloyloxyéthyl triméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolyméristion étant suivie d'une réticulation par un composé à insaturation oléfinique, en particulier le méthylène-bis acrylamide.

On utilise plus particulièrement un copolymère réticulé acrylamide/chlorure de méthacryloyloxyéthyl triméthylammonium (20/80 en poids) sous forme de dispersion contenant 50% en poids dudit copolymère dans de l'huile minérale. Cette dispersion est commercialisée sous le nom de SALCARE SC92 par la Société ALLIED COLLOIDS. On utilise également un homopolymère réticulé du chlorure de méthacryloyloxyéthyl triméthylammonium contenant environ 50% en poids de l'homopolymère dans de l'huile minérale. Cette dispersion est commercialisée sous le nom de SALCARE SC95 par la Société ALLIED COLLOIDS.

D'autres polymères cationiques utilisables conformément à l'invention sont des polyalkylèneimines, en particulier des polyéthylèneimines, des polymères contenant des motifs vinylpyridine ou vinylpyridinium, des condensats de polyamines et d'épichlorhydrine, les polyuréylènes quaternaires et les dérivés de la chitine, les polysiloxanes cationiques.

Les polymères amphotères sont notamment des polymères comportant des motifs A et B répartis statistiquement dans la chaîne polymère où A désigne un motif dérivant d'un monomère comportant au moins un atome d'azote basique et B désigne un motif dérivant d'un monomère acide comportant un ou plusieurs groupements carboxyliques ou sulfoniques ou bien A et B peuvent désigner des groupements dérivant de monomères zwittérioniques de carboxybétaïne; A et B peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien A et B font partie d'une chaîne d'un polymère à motifs éthylène alpha, béta-dicarboxylique dont l'un des groupements carboxylique a été amené à réagir avec une polyamine comportant un ou plusieurs groupements amine primaire ou secondaire ou tertiaire.

De tels polymères sont décrits notamment dans les brevets français FR-2 470 596, FR-2 486 394, FR-2 519 863.

On peut citer en particulier les polymères dérivés du chitosane choisis en particulier parmi ceux décrits dans le brevet français 2 137 684 et comportant des motifs répondant aux formules (VI) :

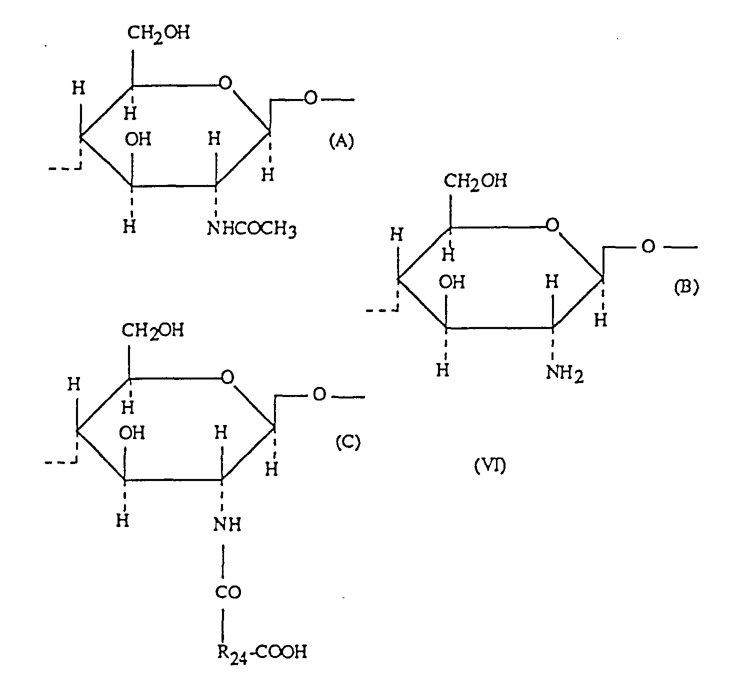

Le motif (A) est présent dans des proportions de 0 à 30%, le motif (B) de 5 à 50%, le motif (C) de 30 à 90% en poids. R₂₄ représente un groupement alkylène linéaire ou ramifié comportant de 2 à 5 atomes de carbone.

Le polymère préféré comporte de préférence 0 à 20% de motif (A), 40 à 50% de motif (B) et 40 à 50% de motif (C) dans lequel R₂₄ désigne un radical alkylène et de préférence -CH₂-CH₂-.

Les polymères dérivés de diallyldialkylammonium et d'un monomère anionique tels que plus particulièrement les polymères comportant 60 à environ 99% en poids d'unité dérivée d'un monomère de diallyldialkylammonium quaternaire, dans lequel les groupements alkyle sont choisis indépendamment parmi les groupements alkyle ayant 1 à 18 atomes de carbone et dans lequel l'anion est dérivé d'un acide ayant une constante d'ionisation supérieure à 10-13 et 1 à 40% en poids de ce polymère d'un monomère anionique choisi parmi les acides acryliques ou méthacryliques, le poids moléculaire de ce polymère étant compris entre environ 50.000 et 10.000.000 déterminé par chromatographie par perméation de gel et décrit en particulier dans la demande EP-A-269 243.

Parmi ces polymères, on peut citer plus particulièrement le copolymère de chlorure de diméthyldiallylammonium et de diéthyldiallylammonium et d'acide acrylique.

Des produits particulièrement préférés sont les produits vendus sous la dénomination "MERQUAT 280" par la Société CALGON sous forme d'une solution aqueuse à 35% de matières actives, ce polymère étant un copolymère de chlorure de diallyldiméthylammonium et d'acide acrylique dans des proportions de 80/20, la viscosité au viscosimètre Brookfield LVF, module 4, étant comprise entre 4.000 et 10.000 cps, le poids moléculaire étant environ égal à 1.300.000.

Les polymères utilisés de façon préférentielle, conformément à l'invention, sont en particulier les dérivés d'éther de cellulose quaternaires tels que les produits vendus sous la dénomination "JR 400" par la Société UNION CARBIDE CORPORATION, les cyclopolymères, en particulier les copolymères du chlorure de diméthyldiallylammonium et d'acrylamide ayant un poids moléculaire supérieur à 500.000, vendus sous les dénominations "MERQUAT 550", "MERQUAT S" par la Société MERCK ou le copolymère de chlorure de diallyldiméthylammonium et d'acide acrylique, commercialisé sous la dénomination "MERQUAT 280" par la Société CALGON.

Les polysaccharides cationiques et plus particulièrement le polymère vendu sous la dénomination "JAGUAR C13S" par la Société MEYHALL.

Les céramides et/ou glycocéramides sont connus en elles-mêmes et sont des molécules naturelles ou synthétiques répondant à la formule générale (VII) : 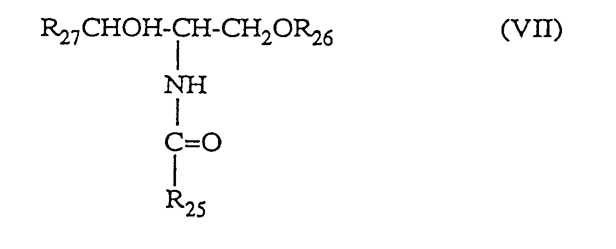 dans laquelle :
R₂₅ désigne un radical alkyle, linéaire ou ramifié, saturé ou insaturé, dérivé d'acides gras en C₁₄-C₃₀, ce radical pouvant être substitué par un groupement hydroxyle en position α ou un groupement hydroxyle en position ω estérifié par un acide gras saturé ou insaturé en C₁₆-C₃₀;
R₂₆ désigne un atome d'hydrogène ou un radical (glycosyle)ₙ, (galactosyle)ₘ ou sulfogalactosyle;
   dans lesquels :
   n est un entier variant de 1 à 4; et
   m est un entier variant de 1 à 8;
R₂₇ désigne un radical hydrocarboné en C₁₅-C₂₆, saturé ou insaturé en position α, pouvant être substitué par un ou plusieurs radicaux alkyle en C₁-C₁₄;
dans le cas des céramides ou glycocéramides naturelles, R₂₇ peut également désigner un radical α-hydroxyalkyle en C₁₅-C₂₆, le groupement hydroxyle étant éventuellement estérifié par un α-hydroxyacide en C₁₆-C₃₀.

Les céramides préférées sont celles décrites par Downing dans Arch. Dermatol, Vol. 123, 1381-1384, 1987, ou celles décrites dans le brevet français FR-2 673 179, dont les structures peuvent être les suivantes :

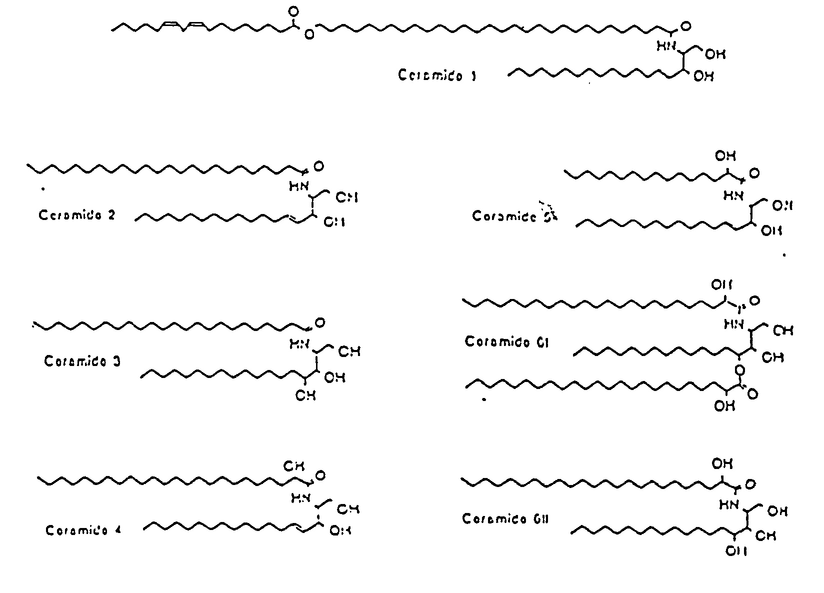

Les céramides plus particulièrement préférés sont les composés de formule (VII), pour lesquels :
R₂₅ désigne un alkyle saturé ou insaturé, dérivé d'acide gras en C₁₆-C₂₂;
R₂₆ désigne hydrogène;
R₂₇ désigne un radical linéaire saturé en C₁₅.

De tels composés sont par exemple :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
ou les mélanges de ces composés.

On utilise également de préférence ceux pour lesquels :
R₂₅ désigne un radical alkyle saturé ou insaturé, dérivé d'acide gras;
R₂₆ désigne galactosyle ou sulfogalactosyle; et
R₂₇ désigne -CH=CH-(CH₂)₁₂-CH₃.

On peut citer le produit constitué d'un mélange de ces composés, vendu sous la dénomination commerciale GLYCOCER par la Société WAITAKI INTERNATIONAL BIOSCIENCES.

Conformément à l'invention, les agents tensio-actifs détergents (anioniques, amphotères, zwitterioniques) sont présents dans des proportions supérieures à 4% et généralement inférieures à 60%.

Les agents tensio-actifs anioniques sont utilisés dans des proportions comprises entre 3 et 50% en poids par rapport au poids total de la composition et de préférence entre 5 et 30% en poids.

Les agents tensio-actifs amphotères ou zwitterioniques sont présents de préférence dans des proportions comprises entre 1 et 50% en poids et de préférence entre 1,5 et 15% en poids.

Les polymères cationiques sont utilisés de préférence dans des proportions de 0,05 à 5% en poids exprimées en matière active et de préférence entre 0,1 et 3% en poids par rapport au poids total de la composition.

Les céramides et/ou glycocéramides sont utilisées de préférence dans des proportions de 0,005% et 5% en poids exprimées en matière active, et de préférence entre 0,01 et 3% en poids par rapport au poids total de la composition.

Le pH des compositions est de préférence compris entre 2 et 9 et en particulier entre 3 et 8. Il est ajusté par des agents alcalinisants ou acidifiants cosmétiquement acceptables.

Les compositions conformes à l'invention peuvent contenir d'autres adjuvants habituellement utilisés dans les compositions de lavage et de conditionnement des cheveux et/ou de la peau.

Ces compositions peuvent contenir notamment des agents tensio-actifs non-ioniques bien connus qui peuvent être choisis parmi les alcools, les alphadiols, les alkylphénols, les acides gras polyéthoxylés, polypropoxylés ou polyglycérolés à chaîne grasse comportant par exemple 8 à 18 atomes de carbone, le nombre de groupements d'oxyde d'éthylène et d'oxyde de propylène pouvant aller en particulier de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras; les amides gras polyéthoxylés ayant de préférence 2 à 30 moles d'oxyde d'éthylène, les amides gras polyglycérolés comportant en moyenne 1 à 5 groupements glycérol et en particulier 1,5 à 4 groupements glycérol; les amines grasses polyéthoxylées ayant de préférence 2 à 3 moles d'oxyde d'éthylène; les esters d'acides gras du sorbitan oxyéthylénés ayant de 2 à 30 moles d'oxyde d'éthylène; les esters d'acides gras du sucrose, les esters d'acides gras du polyéthylèneglycol, les alkylpolyglycosides, les dérivés de N-alkyl glucamine, les oxydes d'amines tels que les oxydes d'alkyl(C₁₀-C₁₄) amines ou les oxydes de N-acylaminopropylmorpholine. Les alkylpolyglycosides et les alcools alphadiols alkylphénols d'acides gras polyglycérolés sont plus particulièrement préféfés.

Les compositions peuvent également contenir des agents épaississants choisis notament parmi l'alginate de sodium, la gomme arabique, les dérivés cellulosiques tels que la méthylcellulose, l'hydroxyméthylcellulose, l'hydroxyéthylcellulose, l'hydroxypropylcellulose, l'hydroxypropylméthylcellulose, la gomme de guar ou ses dérivés, les gommes de xanthane, les scléroglucanes, les acides polyacryliques réticulés, les polyuréthanes, les copolymères à base d'acide ou d'anhydride maléique, les épaississants associatifs porteurs de chaînes grasses de type naturel comme le produit commercialisé sous la dénomination NATROSOL PLUS ou synthétiques comme les produits commercialisés sous la dénomination PEMULEN.

L'épaississement peut également être obtenu par mélange du polyéthylèneglycol et de stéarates ou de distéarates de polyéthylène glycol ou par mélange d'esters phosphoriques et d'amides.

Le milieu cosmétiquement acceptable est de préférence constitué par de l'eau, mais peut également contenir des solvants cosmétiquement acceptables tels que des monoalcools, des polyalcools, des éthers de glycol ou des esters d'acides gras utilisés seuls ou en mélange. Parmi ces solvants, on peut citer plus particulièrement les alcools inférieurs tels que l'éthanol, l'isopropanol, les polyalcools tels que l'éthylèneglycol, le diéthylèneglycol, les éthers de glycol, les alkyléthers de glycol ou de diéthylèneglycol.

Les compositions conformes à l'invention peuvent également contenir des colorants, des agents modificateurs de viscosité, des agents nacrants, des agents hydratants, des agents antipelliculaires, des agents antiséborrhéiques, des filtres solaires, des protéines, des vitamines, des α-hydroxyacides, des sels, des parfums, des agents conservateurs, des séquestrants, des adoucissants, des modificateurs de mousse, des détoxifiants.

Des agents conditionneurs autres que les polymères cationiques peuvent également être utilisés. On peut citer les huiles naturelles hydrogénées ou non, synthétiques ou non, hydrocarbonées, cycliques ou aliphatiques, linéaires ou ramifiées (saturées ou non), les silicones volatiles ou non, organomodifiées ou non, solubles ou non, des huiles perfluorées ou fluorées, les polybutènes et polyisobutènes, des esters gras se présentant sous forme liquide, pâteuse ou solide, les esters d'alcools polyhydriques, des glycérides, les cires naturelles ou synthétiques, des gommes et résines de silicones, ou le mélange de ces différents agents.

Les compositions conformes à l'invention sont plus particulièrement utilisées pour le lavage des cheveux et/ou de la peau et se présentent sous forme de liquides fluides ou épaissis, de gels ou de crèmes.

Elles peuvent être utilisées en l'état ou être diluées avant utilisation. On peut les mettre en oeuvre à l'aide d'un récipient sous pression et elles peuvent être délivrées sous forme liquide, de crème, de gel ou de mousse.

Les exemples qui suivent sont destinés à illustrer l'invention sans pour autant présenter un caractère limitatif.

### EXEMPLES

### EXEMPLES 1 à 7

| | 1 | 2 * | 3 * | 4 | 5 * | 6 | 7 * |
|---|---|---|---|---|---|---|---|
| Lauryl éther sulfate de sodium | 8 g MA | 8 | 8 | 8 | 8 | 8 | 8 |
| Cocoyl bétaïne | 4 g MA | 4 | 4 | 4 | 4 | 4 | 4 |
| MERQUAT 550 | 0,5 g | 0,7 | g | | | | |
| SALCARE SC 92 | | | | 0,5 | 0,7 | | |
| JAGUAR C13S | | | | | | 0,5 | 0,7 |
| CERAMIDE A | 0,2 g | - | 0,7 g | 0,2 g | | 0,2 g | |
| Conservateurs | 0,1 | 0,1 | 0,1 | 0,1 | 0,1 | 0,1 | 0,1 |
| Eau qsp | 100 g | 100 g | 100 g | 100 g | 100 g | 100 g | 100 g |
| pH ajusté NaOH | 6,5 | 6,5 | 6,5 | 6,5 | 6,5 | 6,5 | 6,5 |

| | | | | | | | |
|---|---|---|---|---|---|---|---|
| * Les exemples 2, 3, 5 et 7 ne sont pas conformes à l'invention. | | | | | | | |

On applique sur les cheveux mouillés 1 g de shampooing.

Après un temps de pose de 5 minutes, on procède au rinçage à l'eau courante.

On a demandé à un panel de 10 testeurs de classer les 3 mèches relatives à chaque association en fonction de la facilité de démêlage et de la douceur des cheveux mouillés.

Pour chaque association, les 10 testeurs ont considéré que : les compositions des exemples 1, 4 et 6 étaient plus faciles à démêler et plus douces que les compositions correspondantes ne contenant, soit que le polymère cationique (exemples 2, 5 et 7), soit le céramide (exemple 3).

### Céramide A :

### N-oléoyldihydrosphingosine de formule :

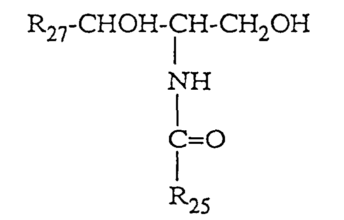

dans laquelle :
R₂₇ = C₁₅H₃₁
R₂₅ = C₁₇H₃₃
**MERQUAT 550** (vendu par la Société Merck) : copolymère de chlorure de diméthyl diallyl ammonium et d'acrylamide
(= polyquatemium-7) (CTFA)
**SALCARE SC 92** (vendu par la Société Allied Colloid)
Dispersion dans l'huile minérale de copolymère réticulé acrylamide/ chlorure de méthacryloyloxyéthyl triméthylammonium, vendu à 50% en copolymère
(= polyquaternium-32) (CTFA)
**JAGUAR C 13 S** (vendu par la Société Meyhall)
Gomme d'hydroxypropyl guar quaternisée par le chlorure de 2,3-époxypropyl triméthylammonium.

### EXEMPLES 8 à 11

| | 8 | 9 * | 10 | 11 * |
|---|---|---|---|---|
| Lauryl éther sulfate de sodium | 8 | 8 | 8 | 8 |
| Cocoyl bétaïne | 4 | 4 | 4 | 4 |
| JR 400 | 0,5 | 0,7 | | |
| POLYMERE A | | | 0,5 | 0,7 |
| CERAMIDE A | 0,2 | | 0,2 | |
| Conservateurs | 0,1 | 0,1 | 0,1 | 0,1 |
| Eau qsp | 100 g | 100 g | 100 g | 100 g |
| pH ajusté | 6,5 | 6,5 | 6,5 | 6,5 |

| | | | | |
|---|---|---|---|---|
| * Les exemples 9 et 11 ne sont pas conformes à l'invention. | | | | |

On procède comme décrit ci-dessus et on constate à nouveau que les mèches traitées avec les shampooings des exemples 8 et 10 sont plus faciles à démêler et plus douces au toucher que les mèches traitées respectivement avec les shampooings des exemples 9, 11 et 3.

### Polymère A :

Polymère comportant des motifs de formule : 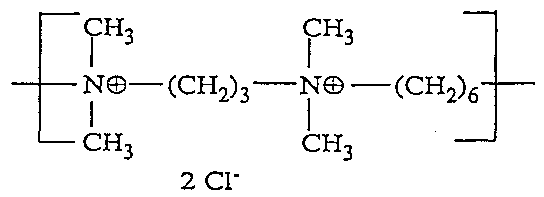 préparé et décrit dans le brevet français n° 2270846.
**JR 400** (vendu par la Société Amerchol)
Polymère d'hydroxyéthylcellulose et d'épichlorhydrine quaternisé avec la triméthylamine
(= polyquatemium-10 - CTFA).

### EXEMPLE 12

On prépare la composition suivante :
- Lauryl éther sulfate de sodium à 2,2 moles d'oxyde d'éthylène en solution aqueuse à 28%, vendu sous la dénomination "EMPICOL ESB/3 FL" par la Société ALBRIGHTetWILSON 10 g MA
- Lauryl éther carboxylate de sodium (C₁₂/C₁₄ 70-30) à 4,5 moles d'oxyde d'éthylène en solution aqueuse à 22%, vendu sous la dénomination "AKYPOSOFT 45 NV" par la Société CHEM'Y 3 g MA
- Cocoamidopropylbétaïne 7 g
- Céramide A 0,4 g
- Polyquaternium 7 0,1 g
- Conservateur, parfum
- Eau qsp 100 g
- pH ajusté à 5 par HCl

Cette composition est utilisée comme gel douche.

### EXEMPLE 13

On prépare la composition suivante :
- Lauryl éther sulfate de sodium à 2,2 moles d'oxyde d'éthylène en solution aqueuse à 28%, vendu sous la dénomination "EMPICOL ESB/3 FL" par la Société ALBRIGHT et WILSON 8 g MA
- Cocoylbétaïne en solution aqueuse à 32% 4 g MA
- Céramide A 0,1 g
- Chlorure hydroxypropyltriammonium de guar 0,4 G
- Conservateurs, parfum
- Eau qsp 100 g
- pH ajusté à 6,9 par HCl

Cette composition est utilisée comme shampooing.

### EXEMPLE 14

On prépare la composition suivante :
- Lauryl éther sulfate de sodium et de magnésium (80/20) à 4 moles d'oxyde d'éthylène, vendu sous la dénomination "EMPICOL BSD" par la Société ALBRIGHT et WILSON à 26% de matière active 10 g MA
- Mélange cocoylamidopropylbétaïne/monolaurate de glycérol (25/5), vendu sous la dénomination "TEGO BETAINE HS" par la Société GOLDSCHMIDT à 30% de matière active 3 g MA
- Céramide A 0,2 g
- Polyquaternium 10 0,5 g
- Conservateurs, parfum
- Eau qsp 100 g
- pH ajusté à 7,2 par NaOH

Cette composition est utilisée comme shampooing.

## Revendications

1. Composition de lavage et de traitement des cheveux et/ou de la peau, caractérisée par le fait qu'elle contient, dans un milieu cosmétiquement acceptable :
- au moins un agent tensio-actif anionique,
- au moins un agent tensio-actif amphotère et/ou zwitterionique, ces agents tensio-actifs étant présents dans des proportions détergentes au moins égales à 4% en poids,
- au moins un polymère à groupements cationiques,
- au moins un céramide et/ou un glycocéramide.

2. Composition selon la revendication 1, caractérisée par le fait que les tensio-actifs anioniques sont choisis parmi les sels alcalins, les sels d'ammonium, les sels d'amines, les sels d'aminoalcools, les sels de magnésium, d'alkylsulfates, d'alkyléthersulfates, d'alkylamidoéthersulfates, d'alkylarylpolyéthersulfates, de monoglycérides sulfates; d'alkylsulfonates, d'alkylamides sulfonates, d'alkylarylsulfonates, d'oléfines sulfonates, de paraffines sulfonates; d'alkylsulfosuccinates, d'alkyléthersulfosuccinates, d'alkylamides sulfosuccinates; d'alkylsulfosuccinamates; d'alkylsulfoacétates; d'alkylphosphates, d'alkylétherphosphates; d'acylsarcosinates, d'acyliséthionates, de N-acyltaurates;
les sels d'acides gras, les acides d'huile de coprah ou d'huile de coprah hydrogénée; des acyl lactylates; des acides alkyl D-galactoside uroniques, leurs sels; les acides d'éthers carboxyliques polyoxyalkylénés.

3. Composition selon la revendication 1 ou 2, caractérisée par le fait que les agents tensio-actifs amphotères ou zwitterioniques sont choisis parmi les dérivés d'amines secondaires ou tertiaires aliphatiques dans lesquels le radical aliphatique est une chaîne linéaire ou ramifiée, comportant 8 à 18 atomes de carbone et contenant au moins un groupement anionique hydrosolubilisant.

4. Composition selon l'une quelconque des revendications 1 à 3, caractérisée par le fait que les polymères à groupements cationiques sont choisis parmi les polymères substantifs comportant des groupements amine primaires, secondaires, tertiaires et/ou quaternaires, faisant partie de la chaîne polymère ou directement reliés à celle-ci, des polymères amphotères ou des protéines quaternisées et ayant un poids moléculaire compris entre 500 et environ 5.000.000.

5. Composition selon la revendication 4, caractérisée par le fait que les polymères à groupements cationiques sont choisis parmi les protéines quaternisées constituées par des polypeptides modifiés chimiquement et portant en bout de chaîne ou greffés sur celle-ci des groupements ammonium quaternaires.

6. Composition selon la revendication 4, caractérisée par le fait que les polymères cationiques sont des polyamines, des polyaminoamides, des polyammonium quaternaires, choisis parmi
(1) les copolymères vinylpyrrolidone/acrylate ou méthacrylate de dialkylaminoalkyle quaternisés ou non;
(2) les dérivés d'éthers de cellulose comportant des groupements ammonium quaternaires;
(3) les dérivés de cellulose cationiques constitués par des copolymères de cellulose ou les dérivés de cellulose greffés par un monomère hydrosoluble d'ammonium quaternaire;
(4) les polysaccharides quaternisés;
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères;
(6) les polyaminoamides solubles dans l'eau, éventuellement réticulés et/ou alcoylés;
(7) les dérivés de polyaminoamides résultant de la condensation de polyalcoylène polyamines avec des acides polycarboxyliques suivie d'une alcoylation par des agents bifonctionnels;
(8) les polymères obtenus par réaction d'une polyalkylène polyamine comportant deux groupements amine primaire et au moins un groupement amine secondaire avec un acide dicarboxylique;
(9) les cyclopolymères ayant un poids moléculaire de 20.000 à 3.000.000, de méthyl diallyl amine ou de diméthyl diallyl ammonium;
(10) les polymères réticulés de chlorure de méthacryloyloxyéthyl triméthylammonium;
(11) les polymères de polyammonium quaternaires;
(12) les homopolymères ou copolymères dérivés d'esters ou d'amides acrylique ou méthacrylique;
(13) les polymères quaternaires de vinylpyrrolidone et de vinylimidazole;
(14) les polyamines;
(15) des polyalkylène imines;
(16) des polymères contenant des motifs vinylpyridine ou vinylpyridinium;
(17) des condensats de polyamines et d'épichlorhydrines;
(18) les polyuréylènes;
(19) les dérivés de la chitine.

7. Composition selon la revendication 4, caractérisée par le fait que les polymères à groupements cationiques sont choisis parmi les polymères amphotères comportant des motifs A et B répartis statistiquement dans la chaîne polymère où A désigne un motif dérivant d'un monomère comportant au moins un atome d'azote basique et B désigne un motif dérivant d'un monomère acide comportant un ou plusieurs groupements carboxyliques ou sulfoniques ou bien A et B peuvent désigner des groupements dérivant de monomères zwittérioniques de carboxy-bétaïne; A et B peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien A et B font partie d'une chaîne d'un polymère à motifs éthylène alpha, béta-dicarboxylique dont l'un des groupements carboxylique a été amené à réagir avec une polyamine comportant un ou plusieurs groupements amine primaire ou secondaire ou tertiaire.

8. Composition selon la revendication 7, caractérisée par le fait que les polymères amphotères sont choisis parmi les polymères dérivés du chitosane, comportant les motifs répondant aux formules (VI) : 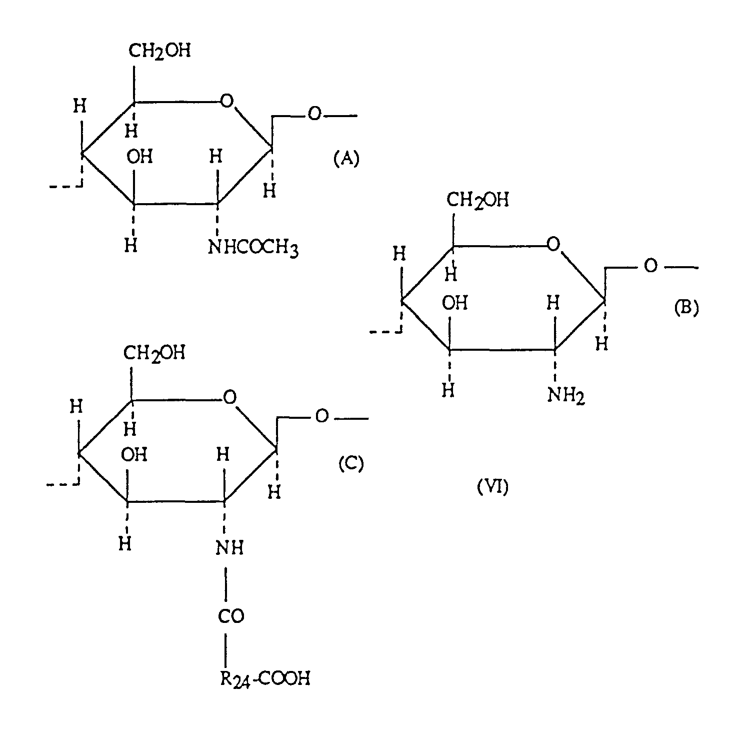 dans laquelle le motif (A) est présent dans des proportions de 0 à 30%, le motif (B) de 5 à 50%, le motif (C) de 30 à 90% en poids, R₂₄ représentant une chaîne alkylène linéaire ou ramifiée de 2 à 5 atomes de carbone, les polymères dérivés de diallyldialkylammonium et d'un monomère anionique sont choisis parmi les acides acryliques ou méthacryliques.

9. Composition selon l'une quelconque des revendications 1 à 8, caractérisée par le fait que les céramides et/ou glycocéramides sont choisies parmi les composés de formule générale : 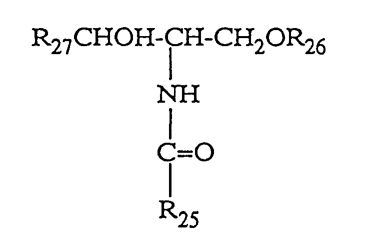 dans laquelle :
R₂₅ désigne un radical alkyle, linéaire ou ramifié, saturé ou insaturé, dérivé d'acides gras en C₁₄-C₃₀, ce radical pouvant être substitué par un groupement hydroxyle en position α ou un groupement hydroxyle en position ω estérifié par un acide gras saturé ou insaturé en C₁₆-C₃₀;
R₂₆ désigne un atome d'hydrogène ou un radical (glycosyle)ₙ, (galactosyle)ₘ ou sulfogalactosyle;
où n est un entier variant de 1 à 4; et m est un entier variant de 1 à 8;
R₂₇ désigne un radical hydrocarboné en C₁₅-C₂₆, saturé ou insaturé en position α, pouvant être substitué par un ou plusieurs radicaux alkyle en C₁-C₁₄;
dans le cas des céramides ou glycocéramides naturelles, R₂₇ peut également désigner un radical α-hydroxyalkyle en C₁₅-C₂₆, un groupement hydroxyle étant éventuellement estérifié par un α-hydroxyacide en C₁₆-C₃₀.

10. Composition selon l'une quelconque des revendications 1 à 9, caractérisée par le fait que les agents tensio-actifs anioniques, amphotères et/ou zwitterioniques sont présents dans des proportions totales inférieures à 60% en poids par rapport au poids de la composition.

11. Composition selon la revendication 10, caractérisée par le fait que les agents tensio-actifs anioniques sont présents dans des proportions comprises entre 3 et 50% en poids, et de préférence entre 5 et 30% en poids par rapport au poids total de la composition.

12. Composition selon la revendication 11, caractérisée par le fait que les agents tensio-actifs amphotères ou zwitterioniques sont présents dans des proportions de 1 à 50% en poids et de préférence de 1,5 à 15% en poids par rapport au poids total de la composition.

13. Composition selon l'une quelconque des revendications 1 à 12, caractérisée par le fait que les polymères cationiques sont présents dans des proportions comprises entre 0,05 et 5% en poids exprimées en matière active et de préférence entre 0,1 et 3% en poids par rapport au poids total de la composition.

14. Composition selon l'une quelconque des revendications 1 à 13, caractérisée par le fait que les céramides et/ou glycocéramides sont présentes dans des proportions comprises entre 0,005 et 5% en poids exprimées en matière active, et de préférence entre 0,01 et 3% en poids par rapport au poids total de la composition.

15. Composition selon l'une quelconque des revendications 1 à 14, caractérisée par le fait que le pH des compositions est compris entre 2 et 9.

16. Composition selon l'une quelconque des revendications 1 à 15, caractérisée par le fait que la composition contient également des agents tensio-actifs non-ioniques.

17. Composition selon l'une quelconque des revendications 1 à 16, caractérisée par le fait que la composition contient des agents épaississants.

18. Composition selon l'une quelconque des revendications 1 à 17, caractérisée par le fait que le milieu cosmétiquement acceptable est constitué par de l'eau, un mélange d'eau et de solvants cosmétiquement acceptables choisis parmi les monoalcools, les polyalcools, les éthers de glycol ou les esters d'acides gras, utilisés seuls ou en mélange.

19. Composition selon l'une quelconque des revendications 1 à 18, caractérisée par le fait que la composition contient également des agents conservateurs, des agents séquestrants, adoucissants, modificateurs de mousse, des colorants, des agents modificateurs de viscosité, des agents nacrants, des agents hydratants, antipelliculaires, antiséborrhéiques, des filtres solaires, des protéines, des vitamines, des α-hydroxyacides, des sels, des détoxifiants, des parfums, ou leurs mélanges.

20. Composition selon l'une quelconque des revendications 1 à 19, caractérisée par le fait que la composition contient également d'autres agents conditionneurs choisis parmi les huiles naturelles hydrogénées ou non, synthétiques ou non, cycliques ou aliphatiques, linéaires ou ramifiées, saturées ou non; les silicones volatiles ou non, organomodifiées ou non, solubles dans le milieu ou non; les huiles fluorées ou perfluorées; des polybutènes, des polyisobutènes; des esters gras, des esters d'alcools polyhydriques; des glycérides; des cires synthétiques ou naturelles, des gommes et résines de silicones; des protéines ou le mélange de ces différents agents.

21. Composition selon l'une quelconque des revendications 1 à 20, caractérisée par le fait qu'elle se présente sous forme de liquide fluide ou épaissi, de gel ou de crème, de mousse, éventuellement conditionnée sous pression.

22. Procédé de lavage et de conditionnement des cheveux, caractérisé par le fait que l'on applique une composition telle que définie dans l'une quelconque des revendications 1 à 21 sur les cheveux et qu'après un éventuel temps de pose, on procède au rinçage.

23. Utilisation de la composition telle que définie dans l'une quelconque des revendications 1 à 21, pour le lavage de la peau ou des cheveux.

## Patentansprüche

1. Zusammensetzung zum Waschen und zur Behandlung der Haare und/oder der Haut, dadurch gekennzeichnet, daß sie in einem kosmetisch akzeptablen Medium enthält:
- mindestens einen anionischen grenzflächenaktiven Stoff,
- mindesten einen amphoteren und/oder zwitterionischen grenzflächenaktiven Stoff, wobei diese grenzflächenaktiven Stoffe in einem Mengenanteil von mindestens 4 Gew.-% vorliegen,
- mindestens ein Polymer mit kationischen Gruppen, und
- mindestens ein Ceramid und/oder Glycoceramid.

2. Zusammensetzung nach Anspruch 1, dadurch gekennzeichnet, daß die anionischen grenzflächenaktiven Stoffe unter den Alkalisalzen, Ammoniumsalzen, Aminsalzen, Aminoalkoholsalzen oder Magnesiumsalzen von Alkylsulfaten, Alkylethersulfaten, Alkylamidoethersulfaten, Alkylarylpolyethersulfaten, Monoglyceridsulfaten, Alkylsulfonaten, Alkylamidsulfonaten, Alkylarylsulfonaten, Olefinsulfonaten, Paraffinsulfonaten, Alkylsulfosuccinaten, Alkylethersulfosuccinaten, Alkylamidsulfosuccinaten, Alkylsulfosuccinamaten, Alkylsulfoacetaten, Alkylphosphaten, Alkyletherphosphaten, Acylsarcosinaten, Acylisethionaten oder N-Acyltauraten; Salzen von Fettsäuren, Säuren von Kopraöl oder hydriertem Kopraöl, Acyllactylaten, Alkyl-D-galactosiduronsäuren und deren Salzen und polyalkoxylierten Carbonsäureethem ausgewählt sind.

3. Zusammensetzung nach Anspruch 1 oder 2, dadurch gekennzeichnet, daß die amphoteren oder zwitterionischen grenzflächenaktiven Stoffe unter den Derivaten von aliphatischen, sekundären oder tertiären Aminen ausgewählt sind, wobei die aliphatische Gruppe eine geradkettige oder verzweigte Gruppe mit 8 bis 18 Kohlenstoffatomen ist, die mindestens eine wasserlösliche anionische Gruppe aufweist.

4. Zusammensetzung nach einem der Ansprüche 1 bis 3, dadurch gekennzeichnet, daß die Polymere mit kationischen Gruppen unter den substantiven Polymeren, die als Teil der Polymerkette oder direkt daran gebunden primäre, sekundäre, tertiäre und/oder quartäre Aminogruppen enthalten, amphoteren Polymeren oder quaternisierten Proteinen mit einem Molekulargewicht von 500 bis etwa 5 000 000 ausgewählt sind.

5. Zusammensetzung nach Anspruch 4, dadurch gekennzeichnet, daß die Polymere mit kationischen Gruppen unter den quaternisierten Proteinen ausgewählt sind, die chemisch modifizierte Polypeptide sind, die am Ende der Kette oder auf die Kette gepfropft quartäre Ammoniumgruppen aufweisen.

6. Zusammensetzung nach Anspruch 4, dadurch gekennzeichnet, daß die Polymere mit kationischen Gruppen Polyamine, Polyaminoamide oder quartäre Polyammoniumverbindungen sind, die ausgewählt sind unter:
(1) Copolymeren von Vinylpyrrolidon und Dialkylaminoalkylacrylat oder Dialkylaminoalkylmethacrylat, die gegebenenfalls quaternisiert sind;
(2) Derivaten von Celluloseethern, die quartäre Ammoniumgruppen enthalten;
(3) Kationischen Cellulosederivaten, wobei es sich um Cellulosecopolymere oder Cellulosederivate handelt, die mit einem wasserlöslichen quartären Ammoniummonomer gepfropft sind;
(4) Quaternisierten Polysacchariden;
(5) Polymeren, die aus Piperazinyleinheiten und zweiwertigen Alkylen- oder Hydroxyalkylengruppen mit geraden oder verzweigten Ketten bestehen, die gegebenenfalls durch Sauerstoffatome, Schwefelatome, Stickstoffatome oder aromatische oder heterocyclische Ringe unterbrochen sind, sowie den Oxidationsprodukten und/oder Quaternisierungsprodukten dieser Polymere;
(6) Wasserlöslichen Polyaminoamiden, die gegebenenfalls vernetzt und/oder alkyliert sind;
(7) Derivaten von Polyaminoamiden, die bei der Kondensation von Polyalkylenpolyaminen mit Polycarbonsäuren und anschließender Alkylierung mit bifunktionellen Mitteln entstehen;
(8) Polymeren, die durch Umsetzung eines Polyalkylenpolyamins, das zwei primäre Aminogruppen und mindestens eine sekundäre Aminogruppe aufweist, mit einer Dicarbonsäure hergestellt sind;
(9) Methyldiallylamin- oder Dimethyldiallylammonium-Cyclopolymeren mit einem Molekulargewicht von 20 00 bis 3 000 000;
(10) Vernetzten Methacryloyloxyethyltrimethylammoniumchloridpolymeren;
(11) Quartären Polyammoniumpolymeren:
(12) Homo- oder Copolymeren, die von Acrylestern, Acrylamiden, Methyacrylestem oder Methacrylamiden abgeleitet sind;
(13) Quartären Polymeren von Vinylpyrrolidon und Vinylimidazol;
(14) Polyaminen;
(15) Polyalkyleniminen;
(16) Polymeren, die Vinylpyridin- oder Vinylpyridiniumeinheiten enthalten;
(17) Kondensaten von Polyaminen und Epichlorhydrinen;
(18) Polyureylenen; und
(18) Chitinderivaten.

7. Zusammensetzung nach Anspruch 4, dadurch gekennzeichnet, daß die Polymere mit kationischen Gruppen unter den amphoteren Polymeren ausgewählt sind, die statistisch in der Polymerkette verteilte Einheiten A und B enthalten, wobei A eine Einheit bedeutet, die von einem Monomer stammt, das mindestens ein basisches Stickstoffatom enthält, und B einen Einheit bedeutet, die von einem sauren Monomer abgeleitet ist, das eine oder mehrere Carboxygruppen oder Sulfonsäuregruppen aufweist, oder A und B Gruppen bedeuten können, die von zwitterionischen Carboxybetainmonomeren abgeleitet sind; A und B können auch kationische Polymerketten bedeuten, die primäre, sekundäre, tertiäre oder quartäre Aminogruppen enthalten, worin mindestens eine Aminogruppe eine Carbonsäuregruppe oder eine Sulfonsäuregruppe trägt, die über eine Kohlenwasserstoffgruppe gebunden ist; oder A und B sind Teil einer Polymerkette mit α,β-Dicarboxyethyleneinheiten, wobei eine der Carbonsäuregruppen mit einem Polyamin umgesetzt wurde, das eine oder mehrere primäre, sekundäre oder tertiäre Aminogruppen trägt.

8. Zusammensetzung nach Anspruch 7, dadurch gekennzeichnet, daß die amphoteren Polymere unter den von Chitosan abgeleiteten Polymeren ausgewählt sind, die Einheiten der folgenden Formel (VI) aufweisen: 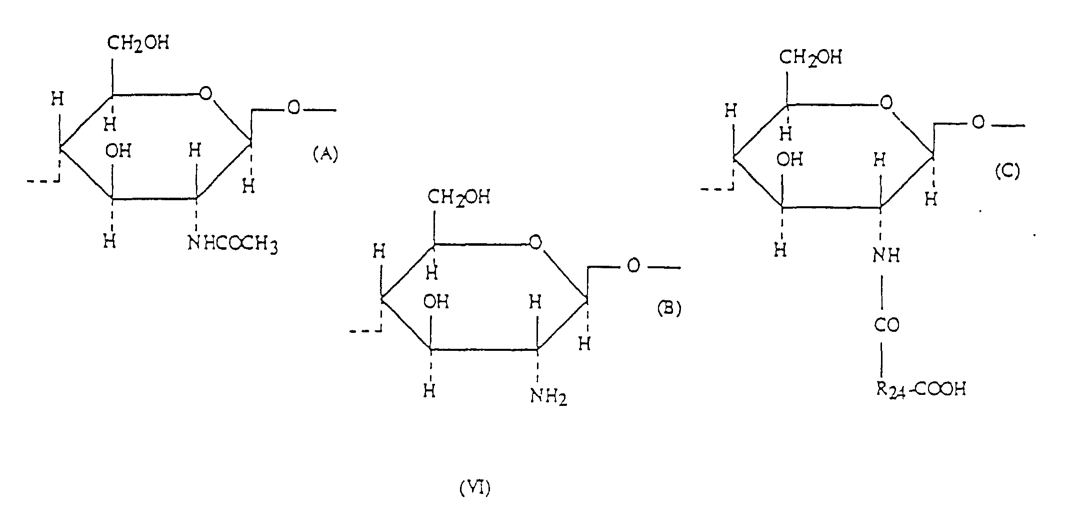 wobei die Einheit (A) in Mengenanteilen von 0 bis 30 Gew.-%, die Einheit (B) von 5 bis 50 Gew.-% und die Einheit (C) von 30 bis 90 Gew.-% vorliegt und R₂₄ eine geradkettige oder verzweigte Alkylengruppe mit 2 bis 5 Kohlenstoffatomen bedeutet; die von Diallyldialkylammonium und einem anionischen Polymer abgeleiteten Polymere sind unter Acryl- oder Methacrylsäure ausgewählt.

9. Zusammensetzung nach einem der Ansprüche 1 bis 8, dadurch gekennzeichnet, daß die Ceramide und/oder Glycoceramide unter den Verbindungen der folgenden allgemeinen Formel ausgewählt sind: 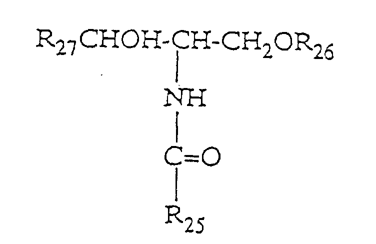 worin bedeuten:
R₂₅ eine geradkettige oder verzweigte, gesättigte oder ungesättigte Alkylgruppe, die von einer C₁₄₋₃₀-Fettsäure abgeleitet ist, wobei die Gruppe in α-Stellung mit einer Hydroxygruppe oder in ω-Stellung mit einer Hydroxygruppe substituiert sein kann, die mit einer gesättigten oder ungesättigten C₁₆₋₃₀-Fettsäure verestert ist;
R₂₆ Wasserstoff oder eine Gruppe (Glykosyl)ₙ, (Galactosyl)ₘ oder Sulfogalactosyl; worin n eine ganze Zahl von 1 bis 4 und m eine ganze Zahl von 1 bis 8 bedeutet;
R₂₇ eine gesättigte oder in α-Stellung ungesättigte C₁₅₋₂₆-Kohlenwasserstoffgruppe, die mit einer oder mehreren C₁₋₁₄-Alkylgruppen substituiert sein kann;
wobei im Falle der natürlichen Ceramide oder Glycoceramide R₂₇ auch eine C₁₅₋₂₆-α-Hydroxyalkylgruppe bedeuten kann, worin eine Hydroxygruppe gegebenenfalls mit einer C₁₆₋₃₀-α-Hydroxysäure verestert ist.

10. Zusammensetzung nach einem der Ansprüche 1 bis 9, dadurch gekennzeichnet, daß die anionischen, amphoteren und/oder zwitterionischen grenzflächenaktiven Stoffe insgesamt in Mengenanteilen unter 60 %, bezogen auf das Gesamtgewicht der Zusammensetzung, vorliegen.

11. Zusammensetzung nach Anspruch 10, dadurch gekennzeichnet, daß die anionischen grenzflächenaktiven Stoffe in Mengenanteilen von 3 bis 50 Gew.-% und vorzugsweise 5 bis 30 Gew.-%, bezogen auf das Gesamtgewicht der Zusammensetzung, vorliegen.

12. Zusammensetzung nach Anspruch 11, dadurch gekennzeichnet, daß die amphoteren oder zwitterionischen grenzflächenaktiven Stoffe in Mengenanteilen von 1 bis 50 Gew.-% und vorzugsweise 1,5 bis 15 Gew.-%, bezogen auf das Gesamtgewicht der Zusammensetzung, vorliegen.

13. Zusammensetzung nach einem der Ansprüche 1 bis 12, dadurch gekennzeichnet, daß die kationischen Polymere in als Wirkstoff ausgedrückten Mengenanteilen von 0,05 bis 5 Gew.-% und vorzugsweise 0,1 bis 3 Gew.-%, bezogen auf das Gesamtgewicht der Zusammensetzung, vorliegen.

14. Zusammensetzung nach einem der Ansprüche 1 bis 13, dadurch gekennzeichnet, daß die Ceramide und/oder Glycoceramide in als Wirkstoff ausgedrückten Mengenanteilen von 0,005 bis 5 Gew.-% und vorzugsweise 0,01 bis 3 Gew.-%, bezogen auf das Gesamtgewicht der Zusammensetzung, vorliegen.

15. Zusammensetzung nach einem der Ansprüche 1 bis 14, dadurch gekennzeichnet, daß der pH-Wert im Bereich von 2 bis 9 liegt.

16. Zusammensetzung nach einem der Ansprüche 1 bis 15, dadurch gekennzeichnet, daß die Zusammensetzung auch nichtionische grenzflächenaktiven Stoffe enthält.

17. Zusammensetzung nach einem der Ansprüche 1 bis 16, dadurch gekennzeichnet, daß die Zusammensetzung Verdickungsmittel enthält.

18. Zusammensetzung nach einem der Ansprüche 1 bis 17, dadurch gekennzeichnet, daß das kosmetisch akzeptable Medium aus Wasser oder einem Gemisch von Wasser und kosmetisch akzeptablen Lösemitteln, wie Monoalkoholen, Polyalkoholen, Glykolethern oder Fettsäureestern oder deren Gemischen, besteht.

19. Zusammensetzung nach einem der Ansprüche 1 bis 18, dadurch gekennzeichnet, daß die Zusammensetzung ferner Konservierungsmittel, Maskierungsmittel, Mittel für die Geschmeidigkeit, Schaummodifikatoren, Färbemittel, Viskositätsmodifikatoren, Perlglanzpigmente, Hydratisierungsmittel, Mittel gegen Schuppen, Antiseborrhöika, Sonnenschutzfilter, Proteine, Vitamine, α-Hydroxysäuren, Salze, Detoxikationsmittel, Parfums oder deren Gemische enthält.

20. Zusammensetzung nach einem der Ansprüche 1 bis 19, dadurch gekennzeichnet, daß die Zusammensetzung auch weitere Konditioniermittel enthält, die unter den natürlichen hydrierten oder nicht hydrierten, synthetischen oder nicht synthetischen, cyclischen oder aliphatischen, geradkettigen oder verzweigten, gesättigten oder ungesättigten Ölen; flüchtigen oder nicht flüchtigen, gegebenenfalls organomodifizierten, in dem Medium löslichen oder nicht löslichen Siliconen; fluorierten oder perfluorierten Ölen; Polybutenen und Polyisobutenen; Fettsäureestern, Estern von mehrwertigen Alkoholen; Glyceriden; natürlichen oder synthetischen Wachsen, Silicongummis und Siliconharzen; Proteinen oder den Gemischen dieser verschiedenen Mittel ausgewählt sind.

21. Zusammensetzung nach einem der Ansprüche 1 bis 20, dadurch gekennzeichnet, daß sie in Form einer fluiden oder dickflüssigen Flüssigkeit, eines Gels, einer Creme oder eines Schaum, die gegebenenfalls unter Druck konfektioniert sind, vorliegt.

22. Verfahren zum Waschen und Konditionieren der Haare, dadurch gekennzeichnet, daß auf das Haar eine Zusammensetzung nach einem der Ansprüche 1 bis 21 aufgetragen und gegebenenfalls nach einer Einwirkzeit gespült wird.

23. Verwendung einer Zusammensetzung nach einem der Ansprüche 1 bis 21 zum Waschen der Haut oder der Haare.

## Claims

1. Hair and/or skin washing and treatment composition, characterized in that it contains, in a cosmetically acceptable medium:
- at least one anionic surface-active agent,
- at least one amphoteric and/or zwitterionic surface-active agent,
these surface-active agents being present in detergent proportions at least equal to 4% by weight,
- at least one polymer containing cationic groups,
- at least one ceramide and/or one glycoceramide.

2. Composition according to Claim 1, characterized in that the anionic surfactants are chosen from alkali metal salts, ammonium salts, amine salts, amino alcohol salts and magnesium salts of alkyl sulphates, alkyl ether sulphates, alkylamido ether sulphates, alkylaryl polyether sulphates, monoglyceride sulphates; alkylsulphonates, alkylamide sulphonates, alkylarylsulphonates, olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl phosphates, alkyl ether phosphates; acyl sarcosinates, acyl isethionates, N-acyltaurates;
fatty acid salts, coconut oil acid or hydrogenated coconut oil acid; acyl lactylates; alkyl-D-galactosiduronic acids and salts thereof; polyoxyalkylenated carboxylic ether acids.

3. Composition according to Claim 1 or 2, characterized in that the amphoteric or zwitterionic surface-active agents are chosen from aliphatic secondary or tertiary amine derivatives in which the aliphatic radical is a linear or branched chain containing 8 to 18 carbon atoms and containing at least one water-soluble anionic group.

4. Composition according to any one of Claims 1 to 3, characterized in that the polymers containing cationic groups are chosen from substantive polymers containing primary, secondary, tertiary and/or quaternary amine groups, which form part of the polymer chain or are directly attached thereto, amphoteric polymers or quaternized proteins, and having a molecular weight between 500 and approximately 5,000,000.

5. Composition according to Claim 4, characterized in that the polymers containing cationic groups are chosen from quaternized proteins consisting of chemically modified polypeptides bearing quaternary ammonium groups at the end of the chain or grafted onto the latter.

6. Composition according to Claim 4, characterized in that the cationic polymers are polyamines, polyamino amides or quaternary polyammoniums, chosen from
(1) quaternized or non-quaternized vinylpyrrolidone/dialkylaminoalkyl acrylate or methacrylate copolymers;
(2) cellulose ether derivatives containing quaternary ammonium groups;
(3) cationic cellulose derivatives consisting of cellulose polymers or of cellulose derivatives grafted with a water-soluble quaternary ammonium monomer;
(4) quaternized polysaccharides;
(5) polymers consisting of piperazinyl moieties and divalent alkylene or hydroxyalkylene radicals containing straight or branched chains, which are optionally interrupted with oxygen, sulphur or nitrogen atoms or with aromatic or heterocyclic rings, as well as the oxidation and/or quaternization products of these polymers;
(6) water-soluble polyamino amides which are optionally crosslinked and/or alkylated;
(7) polyamino amide derivatives resulting from the condensation of polyalkylene polyamines with polycarboxylic acids followed by alkylation of the difunctional agents;
(8) polymers obtained by reaction of a polyalkylene polyamine containing two primary amine groups and at least one secondary amine group with a dicarboxylic acid;
(9) cyclopolymers having a molecular weight of 20,000 to 3,000,000, of methyldiallylamine or of dimethyldiallylammonium;
(10) crosslinked polymers of methacryloyloxyethyltrimethylammonium chloride;
(11) quaternary polyammonium polymers;
(12) homopolymers or copolymers derived from acrylic or methacrylic esters or amides;
(13) quaternary polymers of vinylpyrrolidone and of vinylimidazole;
(14) polyamines;
(15) polyalkylene imines;
(16) polymers containing vinylpyridine or vinylpyridinium moieties;
(17) condensates of polyamines and epichlorohydrin;
(18) polyureylenes;
(19) chitin derivatives.

7. Composition according to Claim 4, characterized in that the polymers containing cationic groups are chosen from amphoteric polymers containing moieties A and B which are statistically distributed in the polymer chain, where A denotes a moiety derived from a monomer containing at least one basic nitrogen atom and B denotes a moiety derived from an acidic monomer containing one or more carboxylic or sulphonic groups or alternatively A and B may denote groups derived from carboxybetaine zwitterionic monomers; A and B may also denote a cationic polymer chain containing primary, secondary, tertiary or quaternary amine groups in which at least one of the amine groups bears a carboxylic or sulphonic group which is attached via a hydrocarbon radical or alternatively A and B form part of a chain of a polymer containing ethylene *α* ,*β*-dicarboxylic moieties in which one of the carboxylic groups has been reacted with a polyamine containing one or more primary or secondary or tertiary amine groups.

8. Composition according to Claim 7, characterized in that the amphoteric polymers are chosen from polymers derived from chitosan, containing moieties corresponding to the formulae (VI) : 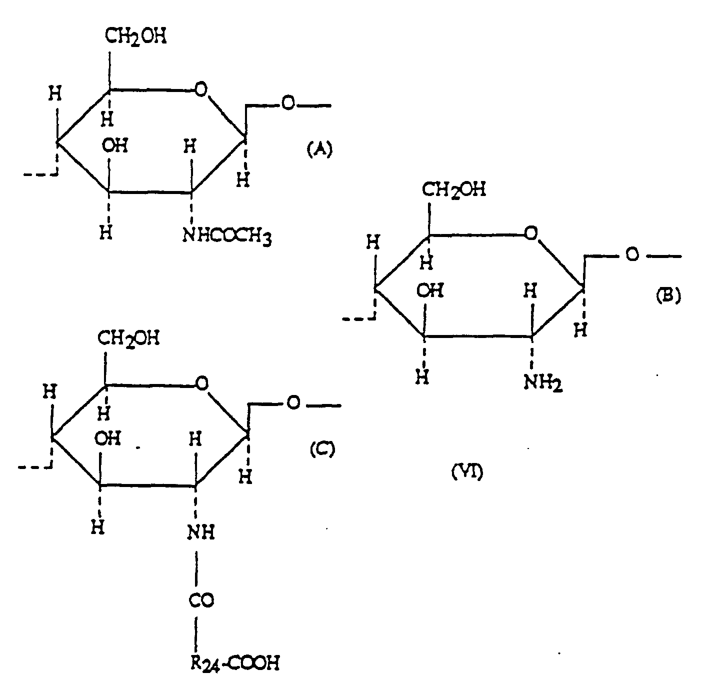 in which the moiety (A) is present in proportions from 0 to 30%, the moiety (B) from 5 to 50%, the moiety (C) from 30 to 90% by weight, R₂₄ representing a linear or branched alkylene chain of 2 to 5 carbon atoms, the polymers derived from diallyldialkylammonium and an anionic monomer are chosen from acrylic acid or methacrylic acid.

9. Composition according to any one of Claims 1 to 8, characterized in that the ceramides and/or glycoceramides are chosen from the compounds of general formula: 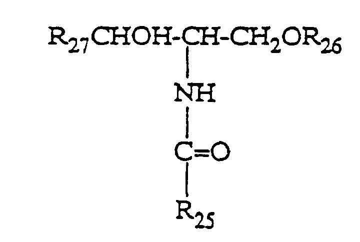 in which
R₂₅ denotes a saturated or unsaturated linear or branched alkyl radical derived from C₁₄-C₃₀ fatty acids, it being possible for this radical to be substituted with a hydroxyl group in the a-position or a hydroxyl group in the ω-position which is esterified with a saturated or unsaturated C₁₆-C₃₀ fatty acid;
R₂₆ denotes a hydrogen atom or a (glycosyl)ₙ, (galactosyl)ₘ or sulphogalactosyl radical;
where n is an integer ranging from 1 to 4; and m is an integer ranging from 1 to 8;
R₂₇ denotes a saturated or unsaturated C₁₅-C₂₆ hydrocarbon radical in the α-position which may be substituted with one or more C₁-C₁₄ alkyl radicals;
in the case of natural ceramides or glycoceramides, R₂₇ may also denote a C₁₅-C₂₆α-hydroxyalkyl radical, a hydroxyl group optionally being esterified with a C₁₆-C₃₀ α-hydroxy acid.

10. Composition according to any one of Claims 1 to 9, characterized in that the anionic, amphoteric and/or zwitterionic surface-active agents are present in total proportions of less than 60% by weight relative to the weight of the composition.

11. Composition according to Claim 10, characterized in that the anionic surface-active agents are present in proportions between 3 and 50% by weight, and preferably between 5 and 30% by weight, relative to the total weight of the composition.

12. Composition according to Claim 11, characterized in that the amphoteric or zwitterionic surface-active agents are present in proportion from 1 to 50% by weight, and preferably from 1.5 to 15% by weight, relative to the total weight of the composition.

13. Composition according to any one of Claims 1 to 12, characterized in that the cationic polymers are present in proportions between 0.05 and 5% by weight, expressed as active material, and preferably between 0.1 and 3% by weight, relative to the total weight of the composition.

14. Composition according to any one of Claims 1 to 13, characterized in that the ceramides and/or glycoceramides are present in proportions between 0.005 and 5% by weight, expressed as active material, and preferably between 0.01 and 3% by weight, relative to the total weight of the composition.

15. Composition according to any one of Claims 1 to 14, characterized in that the pH of the compositions is between 2 and 9.

16. Composition according to any one of Claims 1 to 15, characterized in that the composition also contains nonionic surface-active agents.

17. Composition according to any one of Claims 1 to 17, characterized in that the composition contains thickening agents.

18. Composition according to any one of Claims 1 to 17, characterized in that the cosmetically acceptable medium consists of water or a mixture of water and cosmetically acceptable solvents chosen from monoalcohols, polyalcohols, glycol ethers or fatty acid esters, which are used alone or as a mixture.

19. Composition according to any one of Claims 1 to 18, characterized in that the composition also contains preserving agents, sequestering agents, softening agents, foam modifiers, dyes, viscosity-modifying agents, nacreous agents, hydrating agents, anti-dandruff agents, anti-seborrhoeic agents, sunscreen agents, proteins, vitamins, α-hydroxy acids, salts, detoxifying agents, fragrances or mixtures thereof.

20. Composition according to any one of Claims 1 to 19, characterized in that the composition also contains other conditioning agents chosen from saturated or unsaturated linear or branched cyclic or aliphatic synthetic or non-synthetic, hydrogenated or unhydrogenated natural oils, volatile or non-volatile silicones which may or may not be organically modified and which may or may not be soluble in the medium fluorinated or perfluorinated oils, polybutenes and polyisobutenes, fatty esters, esters of polyhydric alcohols, glycerides, natural or synthetic waxes, silicone gums and resins, proteins or the mixture of these various agents.

21. Composition according to any one of Claims 1 to 20, characterized in that it is in the form of a fluid or thickened liquid, a gel, a cream or a foam, optionally packaged under pressure.

22. Process for washing and conditioning the hair, characterized in that a composition as defined in any one of Claims 1 to 21 is applied to the hair and that after optionally leaving in place for a period of time, the hair is rinsed.

23. Use of the composition as defined in any one of Claims 1 to 21, for washing the skin or the hair.
